(19) Europäisches Patentamt
European Patent Office
Office européen des brevets

(11) Publication number: 0 211 408 A2

# (12) EUROPEAN PATENT APPLICATION

(21) Application number: 86110613.6

(51) Int. Cl.⁴: A 61 K 6/08

(22) Date of filing: 31.07.86

(30) Priority: 02.08.85 JP 171360/85

(43) Date of publication of application: 25.02.87 Bulletin 87/9

(84) Designated Contracting States: DE FR GB

(71) Applicant: DAIKIN INDUSTRIES, LIMITED
Shin-Hankyu Building 12/39, Umeda 1-chome Kita-ku
Osaka-shi Osaka-fu(JP)

(72) Inventor: Yamazaki, Noboru
2-11-7, Seta, Setagaya-ku
Tokyo-to(JP)

(72) Inventor: Kurata, Shigeaki
1-1-5, Izumimachi
Kokubunji-shi Tokyo-to(JP)

(72) Inventor: Ohmori, Akira
3-16-22, Yamatedai
Ibaraki-shi Osaka-fu(JP)

(72) Inventor: Ishiwari, Kazuo
407, 12-ban, Hozumidai
Ibaraki-shi Osaka-fu(JP)

(74) Representative: Schüler, Horst, Dr. European Patent Attorney
Kaiserstrasse 41
D-6000 Frankfurt/Main 1(DE)

## (54) Dental materials.

(57) This invention provides a dental material comprising a polymer having at least about 40% by weight of a structural unit represented by the formula

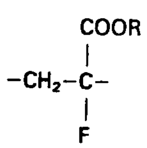

wherein R is aliphatic group having 1 to 5 carbon atoms.

EP 0 211 408 A2

# DENTAL MATERIALS

This invention relates to a dental material which comprises a polymer prepared from acrylate monomer or other monomers and which is useful for producing a restorative appliance or the like.

While a denture plate or the like is preferably made as thin as possible to give the least feeling of incompatibility to the wearer when fitted in the mouth, too thin a denture plate may be often broken when in occlusion, substantial stress is concentratedly applied to a particular part of the denture.

Japanese Unexamined Patent Publications Nos. 81405/1982 and 35505/1982 disclose dental materials comprising polymers prepared from acrylate monomers and useful for a denture, adhesives or the like. However, the dental material described in the former publication has yet to be improved in the mechanical strength required of dentures or the like such as tensile strength, bending strength, etc. And the dental material taught in the latter comprises a polymer consisting mostly of a monomer having no less than 6 vinyl groups to enhance the mechanical strength by cross-linking the polymer, although such monomer is not easy to synthesize.

Our research revealed that a polymer composed of

acrylate with a simple structure having fluorine at the α-position exhibits an outstanding mechanical strength and is useful as a material for dentures or the like. This invention has been accomplished based on this novel finding.

It is an object of the present invention to provide a novel dental material comprising an acrylate polymer.

This invention provides a dental material comprising a polymer having at least about 40% by weight of a structural unit represented by the formula

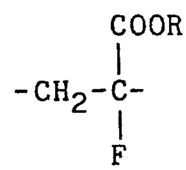

wherein R is aliphatic group having 1 to 5 carbon atoms.

Preferred R group contained in the structural unit of said formula is alkyl group having 1 to 5 carbon atoms. The most preferred alkyl group is methyl.

The polymer of this invention can be prepared by homopolymerizing a monomer represented by the formula

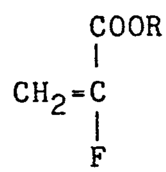

wherein R is as defined above, or copolymerizing at least 40% by weight of the foregoing monomer with not more than

60% by weight of a monomer represented by the formula

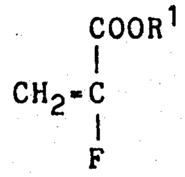

wherein $R^1$ is fluorine-containing aliphatic group having 1 to 5 carbon atoms and/or a monomer represented by the formula

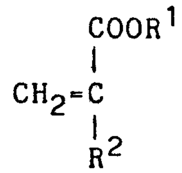

wherein $R^1$ is as defined above and $R^2$ is hydrogen atom or methyl group.

Preferred $R^1$ group in the above structural units is fluorine-containing alkyl group having 1 to 5 cabon atoms and the most preferred fluorine-containing alkyl group is methyl.

Each of these monomers may be a mixture of monomers which fall in the range of the respective formula.

Other monomers can be conjointly used insofar as the monomer used does not impair the properties of the dental material of the invention.

The polymer of the invention can be prepared by polymerizing the monomer(s) by the polymerization method commonly used for polymerizing ethylenically unsaturated

monomers. However, since dentures or the like are made usually based on prosthetist's impression of various shapes obtained from the mouth, it is desired that the polymer be prepared by bulk or mass polymerization method which facilitates the molding or by the polymerization method involving heating of solids and liquids which is frequently used in dental field (e.g. method stated in Japanese Unexamined Patent Publication No. 35505/1982).

According to this invention, polymerization is carried out by adding a polymerization initiator to the monomer(s) or by irradiating the monomer(s) with ultraviolet light or visible light.

Polymerization initiators decomposable around room temperature and above to produce radicals are usually used in the invention. Examples of useful polymerization initiators are tri-n-butylborane or like alkyl metals capable of producing radicals on reaction with oxygen; peroxides such as benzoyl peroxide, acetyl peroxide, lauroyl peroxide, cumene hydroperoxide, methyl ethyl ketone peroxide, t-butyl peroxide benzoate and the like; mixtures of such peroxide with a polymerization accelerator such as tertiary amine, cobalt salt of naphthenic acid or octenoic acid, transition metal ion, p-toluenesulfonic acid and amine salt of sulfinic acid; etc. The polymerization initiator and polymerization

accelerator are each used usually in an amount of about 0.1 to about 2.5 parts by weight per 100 parts by weight of the total monomer(s).

When polymerization is conducted by radiation of the monomer(s) with UV light or visible light according to this invention, a photosensitizer is generally used. Examples of useful photosensitizers are benzophenone, nitrofluorene, 5-nitroacenaphthene and the like for UV light radiation, and camphorquinone for visible light radiation.

The dental materials of the invention can contain additives commonly used for dental plastics materials such as silica, alumina, silicon nitride or the like.

The dental materials of the invention are higher in compressive strength, indirect tensile strength, bending strength, flexural modulus and hardness than conventional dentures comprising polymers prepared from acrylic monomer(s) having a simple molecular structure like the monomers to be used in this invention, hence excellent.

The dental materials of this invention, because of their excellence in mechanical strength, water resistance, coefficient of thermal expansion and other properties, are usable for producing composite restorative

materials, denture base materials, hard crown and bridge resins, cementing materials, orthodontic bonding agents, cavity varnishes, pit and fissure sealants and the like.

This invention will be described below in greater detail with reference to Examples and Comparison Example.

Example 1 and Comparison Example

A 0.5 part quantity by weight of benzoyl peroxide was added to 100 parts by weight of methyl α-fluoroacrylate (Example 1) or methyl methacrylate (Comparison Example). The mixture was fully stirred, placed into a glass tube having an inside diameter of 4 mm (6 or 10 mm) and maintained at 55°C for 24 hours and further at 100°C for 24 hours to polymerize each acrylate.

The polymers thus obtained were tested for properties by the following methods to confirm that the polymer prepared in Example 1 is useful as a dental material. Table 1 below shows the results.

Test for compressive strength

Using as a specimen a polymer 4 mm in diameter and 8 mm in length and retained in water at 37°C for 24 hours, the test was conducted by the method of JIS K 7208 with use of an autographic device, Model "IS-500" (tradename for a product of Shimazu Seisakusho Ltd., Japan) operated at a crosshead speed of 2.5 mm/min. The

value of yield point of the obtained stress-strain curves was calculated as the strength.

Test for indirect tensile strength

Using as a specimen a polymer 6 mm in diameter and 5 mm in length and retained in water at 37°C for 24 hours, the test was conducted by the diametral method at the same crosshead speed as above.

Test for bending strength and flexural modulus

Using as a specimen a polymer 4 mm in diameter and 25 mm in length and retained in water at 37°C for 50 hours, the test was conducted by the three-point bending test method (20 mm in distance between the supported ends) at a crosshead speed of 1 mm/min.

Test for hardness

Using as a specimen the polymer 10 mm in diameter and 1 mm in length and retained in water at 37°C for 50 hours, the test was conducted by a microhardness tester (Model "DMH-2", product of Matsuzawa Seiki Kabushiki Kaisha, Japan) operated under a load of 25 g for a load duration of 5 seconds. The measurement was in terms of Vickers hardness.

Examples 2 to 6

The same procedure as in Example 1 was repeated with the exception of using methyl α-fluoroacrylate and a monomer (3FFA) represented by the formula:

$CH_2{=}C(F)COOCH_2CF_3$ in the proportions specified below in Table 2, thereby producing a polymer.

The polymers thus obtained were subjected to the same tests as above. Table 2 below shows the results.

Example 7

A 18 g quantity of the poly(methyl α-fluoroacrylate) prepared in Example 1 was finely divided. Thereto was added 14 ml of a mixture of methyl α-fluoroacrylate and α-fluoroacrylic acid-α'-trifluoroethyl in a ratio of 6/4 with 0.5% by weight of benzoyl peroxide, whereby the fine particles were swollen into an elastic soft mass. The mass thus obtained was pressed into a mold produced for a maxillary full denture and was maintained therein at 60°C for 8 hours and further at 100°C for 15 hours. Then the mass was left to stand for cooling and withdrawn from the mold. The denture thus prepared was free from feeling of incompatibility when fitted into the mouth and was found to have a high mechanical strength.

Table 1

| | Compressive strength ($kgf/cm^2$) | Indirect tensile strength ($kgf/cm^2$) | Bending strength ($kgf/cm^2$) | Flexural modulus ($X\ 10^2 kgf/cm^2$) | Hardness (Hv) |
|---|---|---|---|---|---|
| Example 1 | 1614 | 524 | 2453 | 327 | 21.6 |
| Comp. Example | 1162 | 362 | 1687 | 209 | 19.4 |

Table 2

| Example | 3FFA in polymer (wt %) | Compressive strength ($kgf/cm^2$) | Indirect tensile strength ($kgf/cm^2$) | Bending strength ($kgf/cm^2$) | Flexural modulus ($\times 10^2 kgf/cm^2$) | Hardness (Hv) |
|---|---|---|---|---|---|---|
| 2 | 10 | 1652 | 509 | 2342 | 312 | 21.5 |
| 3 | 20 | 1547 | 486 | 2246 | 307 | 22.4 |
| 4 | 30 | 1368 | 435 | 2152 | 295 | 21.5 |
| 5 | 40 | 1333 | 406 | 2094 | 277 | 19.9 |
| 6 | 50 | 1215 | 369 | 1972 | 255 | 19.6 |

CLAIMS

1. A dental material comprising a polymer having at least about 40% by weight of a structural unit represented by the formula

$$\begin{array}{c} \phantom{-CH_2-}COOR \\ \phantom{-CH_2-}| \\ -CH_2-C- \\ \phantom{-CH_2-}| \\ \phantom{-CH_2-}F \end{array}$$

wherein R is aliphatic group having 1 to 5 carbon atoms.

2. A dental material as defined in claim 1 wherein the aliphatic group is alkyl group having 1 to 5 carbon atoms.

3. A composite restorative material comprising the dental material as defined in claim 1.

4. A denture base material comprising the dental material as defined in claim 1.

5. A hard crown and bridge resin comprising the dental material as defined in claim 1.

6. A cementing material comprising the dental material as defined in claim 1.

7. An orthodontic bonding agent comprising the dental material as defined in claim 1.

8. A cavity varnish comprising the dental material as defined in claim 1.

9. A pit and fissure sealant comprising the dental material as defined in claim 1.